# EUROPEAN PATENT SPECIFICATION

(11) **EP 3 052 072 B1**
(45) Date of publication and mention of the grant of the patent: **18.04.2018**
(21) Application number: 14780804.2
(22) Date of filing: 25.09.2014
(51) Int. Cl.: A61K 8/25, A61K 8/29, A61Q 17/04, A61K 8/02, A61K 8/27

(54) **TOPICAL SUN SCREEN COMPOSITIONS COMPRISING TITANIUM DIOXIDE AND SILICA**
TOPISCHE SONNENSCHUTZZUSAMMENSETZUNGEN ENTHALTEND TITANDIOXID UND SILICA
COMPOSITION POUR LA PROTECTION CONTRE LE SOLEIL COMPRENANT DE DIOXYDE DE TITANE ET DE SILICE

(30) Priority: 30.09.2013 EP 13186725; 30.09.2013 EP 13186729
(43) Date of publication of application: 10.08.2016
(73) Proprietor: DSM IP Assets B.V., 6411 TE Heerlen (NL)
(72) Inventor: HUEBER, Aline, CH-4002 Basel (CH); SAECKER, Christine, CH-4002 Basel (CH)
(74) Representative: Berg, Katja
(86) International application number: PCT/EP2014/070561
(87) International publication number: WO 2015/044306

(56) References cited:
- EP-A1- 0 988 853
- US-A- 5 968 531
- US-A- 6 004 567
- US-A1- 2003 219 391
- US-A1- 2006 045 890
- US-A1- 2009 252 774

## Description

The present invention relates to a topical composition comprising (1) uncoated, highly porous silica beads having a particle size Dᵥ0 of greater 0.3 µm, a Dᵥ100 of less than 35 µm, a Dᵥ50 selected in the range of 8 to 15 µm and an oil absorption capacity selected in the range of 1.2-2.5 cc/g and (2) a metal oxide selected from microfine double coated titanium dioxide and/ or microfine coated zinc oxide.

Inorganic UV-filter substances such as microfine coated titanium dioxide and zinc oxide are widely used in sunscreens. These inorganic UV-filters, however, have the tendency of leaving a white film on the skin during and after application which is highly unwanted. Thus, there is an ongoing need for ingredients which are able to reduce this whitening effect.

Surprisingly it has been found that the use of specific highly porous silica beads in a topical composition comprising microfine double coated titanium dioxide or microfine coated zinc oxide significantly reduced the whitening effect thereof.

Thus, the present invention relates in a first aspect to topical compositions comprising (1) uncoated, highly porous silica beads having a particle size Dᵥ0 of greater 0.3 µm, a Dᵥ100 of less than 35 µm, a Dᵥ50 selected in the range of 8 to 15 µm and an oil absorption capacity selected in the range of 1.2-2.5 cc/g and a metal oxide selected from microfine double coated titanium dioxide and/ or microfine coated zinc oxide.

Another subject matter of the invention is directed to a method of reducing the whitening effect of a metal oxide selected from microfine double coated titanium dioxide and/ or microfine coated zinc oxide in a topical composition, said method comprising the step of adding to the topical composition uncoated, highly porous silica beads having a particle size Dᵥ0 of greater than 0.3 µm, a Dᵥ100 of less than 35 µm, a Dᵥ50 selected in the range of 8 to 15 µm and an oil absorption capacity selected in the range of 1.2-2.5 cc/g and appreciating the effect.

In a further embodiment the invention relates to the use of uncoated, highly porous silica beads having a particle size Dᵥ0 of greater than 0.3 µm, a Dᵥ100 of less than 35 µm, a Dᵥ50 selected in the range of 8 to 15 µm and an oil absorption capacity selected in the range of 1.2-2.5 cc/g to reduce the whitening effect of a metal oxide selected from microfine double coated titanium dioxide and/ or microfine coated zinc oxide in a topical composition.

In all embodiments of the present invention, the amount of silica beads is preferably selected in the range of 0.5 to 10 wt.-%, more preferably in the range of 1 to 5 wt.-%, and most preferably in the range of 2 to 4 wt.-% based on the total weight of the composition.

The uncoated, porous silica beads used according to the present invention may be prepared from sodium silicate by emulsion polymerization according to standard procedures such as e.g. via the sol-gel method.

The particle size of the beads according to the invention (in volume %) is determined by a Coulter LS13320 or Malvern Mastersizer 2000 according to standard methods in the art. In number % the average particle size Dₙ50 ranges from 8 to 15 µm, preferably from 9 to 15 µm.

The oil absorption capacity (cc/g) [also often referred to as (cm³/g)] refers to the amount of paraffin (in cc) absorbed by a specified amount (g) of the beads, i.e. the amount until the loose and dry powder disappears.

The oil absorption capacity as referred to in the present invention is determined at 23°C by weighting 2g of the respective beads into a 20 ml beaker glass. Then liquid paraffin (Paraffinum Perliquidum PH.EUR. CAS 8042-47-5) is added. After addition of 4 to 5 drops of paraffin to the powder, mixing is performed using a spatula, and addition of paraffin is continued until conglomerates of oil and powder have formed. From this point, the paraffin is added one drop at a time and the mixture is then triturated with the spatula. The addition of oil is stopped when the loose and dry powder completely disappears and a highly viscous white to transparent homogeneous gel is obtained. The oil absorption capacity (cc/g) is then calculated by the volume of paraffin used (in cc) per g of the respective beads.

In all embodiments of the present invention the oil absorption capacity of the silica beads is preferably selected in the range of 1.2 to 2.0 cc/g, more preferably in the range of 1.3 to 1.8 cc/g.

Suitable porous silica beads according to the present invention are e.g. obtainable as VALVANCE™ *Touch* 210 at DSM Nutritional Products Ltd Kaiseraugst.

In all embodiments of the present invention, the amount of the microfine double coated titanium dioxide in the topical composition is preferably selected in the range of 0.5 to 15 wt.-%, more preferably in the range of 1 to 10 wt.-%, and most preferably in the range of 2 to 5 wt.-% based on the total weight of the composition.

In all embodiments of the present invention, the amount of the microfine coated zinc oxide in the topical composition is preferably selected in the range of 0.5 to 25 wt.-%, more preferably in the range of 1 to 20 wt.-%, such as in the range of 1 to 15 wt.-%, and most preferably in the range of 2 to 10 wt.-% such as in particular in the range of 2 to 5 wt.-% based on the total weight of the composition.

In a particular embodiment of the invention, the microfine double coated titanium dioxide has an inner inorganic silica coating and an outer organic coating.

Such titanium dioxides nanoparticles can e.g. be prepared according to the process described in example 1 of EP-444798. The inner coating of the titanium dioxide particle with inorganic silica can be prepared according to the state of the art as e.g. described in EP-44515, EP-988853, EP-1284277, EP-0988853, and US-5562897, JP-2000319128.

The surface of the titanium dioxide can be pretreated before the coating in order to additionally reduce the surface activity. Such pretreatments are well known to a person skilled in the art and can be performed e.g. with (a) fluoro acids selected from H₂SiF₆, H₂TiF₆, H₂ZrF₆, H₂HfF₆, H₂GeF₆, H₂SnF₆, and/or HBF₄; (b) water-soluble carboxylic acid containing ≥2 hydroxyl groups per carboxyl group in each acid mol.(esp. gluconic acid); (c) water-soluble salts of such carboxylic acids; (d) source of phosphate ions, esp. H₃PO₄ and/or phosphate salts and/ or organophosphoric acids and their salts; (e) inorganic acid such as H₂SO₄, HNO₃3, H₃PO₄, hydrobromic, hydroiodic and or perchloric acid (f) organic component(s) selected from tannins and/or amino-phenolic polymers; and (h) optional oxide, hydroxide.

Preferably, the inner coating layer consists of a minimum of 0.5 wt.-% of inorganic silica (based on titanium dioxide). More, preferably the inner coating layer consists of 0.5 wt.-% to 50 wt.-%, most preferably of 1 wt.-% to 20 wt.-% of inorganic silica (based on titanium dioxide).

In another embodiment the titanium dioxide has an inner alumina (aluminium oxide) coating. The inner coating layer consists of a minimum of 0.5 wt.-% of aluminium oxide (based on titanium dioxide). More, preferably the inner coating layer consists of 0.5 wt.-% to 50 wt.-%, most preferably of 1 wt.-% to 20 wt.-% of aluminium oxide (based on titanium dioxide).

The outer coating is preferably selected from the class of organic coatings such e.g. silicone oils (e.g. simethicones, methicones, dimethicones, polysilicone-15), alkyl silanes (e.g. octyl tri(m)ethoxy silane), olefinic acids (e.g. stearic acid), or polyols (e.g. glycerol) and can be applied to the titanium dioxide particle by methods known to a person skilled in the art e.g. described in FI57124. Preferably the outer coating is selected from the group consisting of simethicone, methicone, dimethicone, polysilicones-15, stearic acid and octyl trimethoxy silane. Most preferably the outer coating is dimethicone. The amount of the outer coating layer is at least 0.25 wt.-% based on the titanium dioxide. Preferably the amount of the outer coating is selected in the range of 0.5 wt.-% to 50 wt.-%, most preferably in the range of 0.5 wt.-% to 10 wt.-% based on the titanium dioxide.

In all embodiments of the present invention, the microfine double coated titanium dioxide preferably has a titanium dioxide content selected in the range of 70-95 wt.-% and a silicon dioxide content selected in the range of 5-20 wt.-%, such as preferable a titanium dioxide content selected in the range of 80-90 wt.-% and a silicon dioxide content selected in the range of 10 to 15 wt.-%, with the proviso that the total content of titanium dioxide and silicone dioxide is selected in the range of 90 - 100 wt.-%.

The particle size of the titanium dioxide is not particularly limited. All particle sizes which are principally useful for incorporating into sunscreen compositions can be used in the topical compositions according to the present invention. However, in all embodiments of the invention the primary particle size of the titanium dioxide (i.e. TiO₂ w/o any coating) is preferably selected in the range of 2 to 100 nm, more preferably in the range of 5 to 50 nm and the secondary particle size (i.e. TiO₂ with double coating) is preferably selected in the range of 0.05 and 50 µm, preferably in the range of 0.1 and 1 µm.

The crystalline form of the titanium dioxide may be of any crystal or amorphous type. For example, titanium dioxide may be any type of amorphous, rutil, anastase, brookite or a mixture thereof. Preferably, the crystalline form of the titanium dioxide is rutil.

Particularly suitable titanium dioxide according to the present invention contains a rutil-type titanium dioxide (TiO₂) core with a double coating of silica (inner coating) and dimethicone (outer coating) and has titanium dioxide content selected in the range of 82-87 wt.-% and a silicon dioxide content selected in the range of 10.5 to 14.5 wt.-%, which is commercially available as PARSOL® TX (INCI: titanium dioxide, silica, dimethicone) at DSM nutritional products Ltd.

Suitable double coated titanium dioxide grades having an inner alumina coating and an outer stearic acid coating are e.g. commercially available as Micro Titanium dioxide MT-01 at Tayca or TTO-55(C) at Ishihara Sangyo Kaisha. Also suitable is double coated titanium dioxide having an inner alumina coating and an outer simethicone coating e.g. commercially available as Eusolex T-2000 at EMD chemicals Inc./Rona.

In a particular embodiment of the invention, the microfine coated zinc oxide has one organic coating such as dimethicone, octyl tri(m)ethoxy silane (also known as tri(m)ethoxycaprylylsilane) or dimethoxydiphenylsilanetriethoxycaprylylsilane cross-polymer.

Particularly suitable zinc oxides according to the present invention encompass Z-COTE HP1® (ZnO coate with triethoxycaprylylsilane) or Z-COTE MAX (ZnO coated with dimethoxydiphenylsilanetriethoxycaprylylsilane cross-polymer) which are commercially available at BASF. Alternatively Zano® 10 Plus (ZnO coated with octyl triethoxy silane) could be used which is available at Umicore. Alternatively, ZinClear-IM™ which has an average particle size of >1.0 micron and is hydrophobically modified may be used. It is available in the form of a dispersion using common cosmetic emollients, such as C₁₂-C₁₅ alkyl benzoate or caprylic/capric triglycerides.

The particle size of the zinc oxide is not particularly limited. All particle sizes which are principally useful for incorporating into sunscreen compositions can be used in the topical compositions according to the present invention. However, in all embodiments of the invention the particle size of the coated zinc oxide is preferably selected in the range of 10 to 200 nm.

In a particular embodiment the topical compositions according to the present invention comprise as metal oxide either double coated titanium dioxide or microfine coated zinc oxide in the absence of any further microfine metal oxide(s).
The term "topical" is understood here to mean external application to keratinous substances, which are in particular the skin, scalp, eyelashes, eyebrows, nails, mucous membranes and hair, most preferred is the application to the skin.

As the compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term cosmetically acceptable carrier refers to all carriers and/or excipients and/or diluents conventionally used in cosmetic compositions.

Preferred topical compositions according to the invention are skin care preparations such as in particular sun care preparations or functional preparations.

Examples of skin care preparations are, in particular, light protective preparations (sunscreens, sun care preparations), anti-ageing preparations, preparations for the treatment of photo-ageing, body oils, body lotions, body gels, treatment creams, skin protection ointments, skin powders, moisturizing gels, (moisturizing) sprays, face and/or body moisturizers, skin-tanning preparations (i.e. compositions for the artificial/sunless tanning and/or browning of human skin), as well as skin lightening preparations as well as BB and CC Creams.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment the topical compositions according to the invention are skin care preparations, such as (body) milks, lotions, sprays, hydrodispersions, foundations, creams, creamgels, serums, toners or gels.

The topical compositions according to the present invention may be in the form of a suspension or dispersion in solvents or fatty substances, or alternatively in the form of an emulsion or micro emulsion (in particular of oil-in-water (O/W) or water-in-oil (W/O)type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, in the form of sticks, masks or as sprays.

In one embodiment, the topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the topical composition according to the invention is an O/W emulsion, then it contains advantageously at least one O/W- or Si/W-emulsifier selected from the list of PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, Glyceryl Stearate Citrate, Glyceryl Stearate SE (self-emulsifying), stearic acid, salts of stearic acid, polyglyceryl-3-methylglycosedistearate. Further suitable emulsifiers are phosphate esters and the salts thereof such as cetyl phosphate (Amphisol® A), diethanolamine cetyl phosphate (Amphisol®DEA), potassium cetyl phosphate (Amphisol® K), sodiumcetearylsulfat, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, Cetearyl Glucoside, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

The at least one O/W, respectively Si/W emulsifier is preferably used in an amount of 0.5 to 10 wt.-%, in particular in the range of 0.5 to 6 wt.-% such as more in particular in the range of 0.5 to 5 wt.-% such as most in particular in the range of 1 to 4 wt.-%, based on the total weight of the composition.

Particular suitable O/W emulsifiers according to the present invention encompass phosphate esters emulsifier of formula (II) 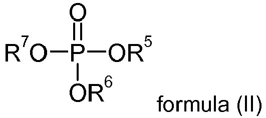 wherein R⁵, R⁶ and R⁷ may be hydrogen, an alkyl of from 1 to 22 carbons, preferably from 12 to 18 carbons; or an alkoxylated alkyl having 1 to 22 carbons, preferably from 12 to 18 carbons, and having 1 or more, preferably from 2 to 25, most preferably 2 to 12, moles ethylene oxide, with the provision that at least one of R⁵, R⁶ and R⁷ is an alkyl or alkoxylated alkyl as previously defined but having at least 6 alkyl carbons in said alkyl or alkoxylated alkyl group.
Monoesters in which R⁵ and R⁶ are hydrogen and R⁷ is selected from alkyl groups of 10 to 18 carbons and alkoxylated fatty alcohols of 10 to 18 carbons and 2 to 12 moles ethylene oxide are preferred. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate.

A particular O/W emusifier to be used in the topical compositions according to the invention is potassium cetyl phosphate e.g. commercially available as Amphisol® K at DSM Nutritional Products Ltd Kaiseraugst.

Further suitable O/W emulsifiers are polyethyleneglycol (PEG) esters or diesters such as e.g. [INCI Names] PEG-100 Stearate, PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate.
Particularly preferred according to the present invention is PEG-100 Stearate sold under the tradename Arlacel™ 165 (INCI Glyceryl Stearate (and) PEG-100 Stearate) by Croda.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

In a particular embodiment, the invention relates to topical compositions with all the definitions and preferences given herein in the form of O/W emulsions comprising an oily phase dispersed in an aqueous phase in the presence of an O/W emulsifier wherein the O/W emulsifier is selected from the group consisting of potassium cetyl phosphate, glyceryl stearate (and) PEG-100 Stearate, cetearyl olivate and sorbitan olivate as well as mixtures thereof.

In another particular embodiment, the invention relates to topical compositions in the form of W/O emulsions comprising an aqueous phase dispersed in an oily phase in the presence of a W/O emulsifier.

Suitable W/O emulsifiers encompasse polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Particularly preferred according to the present invention are W/O emulsions wherein the W/O emulsifier is Polyglyceryl-2 Dipolyhydroxystearate.

The topical compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 5 wt.-%, such as most in particular in the range of 1 to 3 wt.-%, based on the total weight of the composition. Particular suitable co-surfactants are selected from the list of alkyl alcohols such as cetyl alcohol (Lorol C16, Lanette 16) cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof.

The topical compositions in form of O/W or W/O emulsions according to the invention can be provided, for example, in the form of serum, milk, emulsion spray, lotion or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, sun protection and the like).

According to an advantageous embodiment of the invention the topical compositions constitute cosmetic composition and are intended for topical application to the skin.

The topical cosmetic compositions of the invention can also contain usual cosmetic adjuvants, additives, carriers, excipients or diluents conventionally used in cosmetic compositions such as preservatives/antioxidants, fatty substances/oils, water, organic solvents, silicones, thickeners, softeners, emulsifiers, antifoaming agents, aesthetic components such as fragrances, surfactants, fillers, chelating and/ or sequestering agents, anionic, cationic, nonionic or amphoteric polymers or mixtures thereof, propellants, acidifying or basifying agents, dyes, colorings/colorants, abrasives, absorbents, essential oils, sensory modifiers, astringents, or any other ingredients usually formulated into such compositions. Such cosmetic ingredients commonly used in the skin care industry, which are suitable for use in the compositions of the present invention, are e.g. described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

In accordance with the present invention, the topical compositions according to the invention may further comprise cosmetically active ingredients such as skin lightening agents; agents for the prevention of skin tanning; agents for the treatment of hyperpigmentation; agents for the prevention or reduction of acne, wrinkles, lines, atrophy and/or inflammation; anti-cellulites and slimming (e.g. phytanic acid) agents, firming agents, moisturizing and energizing agents, self-tanning agents, soothing agents, as well as agents to improve elasticity and skin barrier and/or further UV-filter substances as well as further pigments or nanopigments, e.g. those suited for providing a photoprotective effect by physically blocking out ultraviolet radiation The necessary amounts of such skin active or protecting agents can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

Preferably, the topical compositions according to the invention comprise further UV filter substances which are preferably selected from conventional UVA and/or UVB and/ or broad spectrum UV-filter substances known to be added into topical compositions such as cosmetic or dermatological sun care products. Such UV-filter substances comprise all groups which absorb light in the range of wavelengths 400 nm to 320 nm (UVA) and 320 nm to 280 nm (UVB) or of even shorter wavelengths (UVC) and which are or can be used as cosmetically acceptable UV-filter substances. Such UV-filter substances are e.g. listed in International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Suitable UV-filter substances may be organic or inorganic compounds. Exemplary organic UV-filter substances encompass e.g. acrylates such as e.g. 2-ethylhexyl 2-cyano-3,3-diphenylacrylate (octocrylene, PARSOL® 340 by DSM Nutritional Products Ltd), ethyl 2-cyano-3,3-diphenylacrylate; Camphor derivatives such as e.g. 4-methyl benzylidene camphor (PARSOL® 5000 by DSM Nutritional Products Ltd), 3-benzylidene camphor, camphor benzalkonium methosulfate, polyacrylamidomethyl benzylidene camphor, sulfo benzylidene camphor, sulphomethyl benzylidene camphor, terephthalylidene dicamphor sulfonic acid (Mexoryl® SX); Cinnamate derivatives such as e.g. ethylhexyl methoxycinnamate (PARSOL® MCX by DSM Nutritional Products Ltd), ethoxyethyl methoxycinnamate, isoamyl methoxycinnamate as well as cinnamic acid derivatives bond to siloxanes; p-Aminobenzoic acid derivatives such as e.g. p-aminobenzoic acid, 2-ethylhexyl p-dimethylaminobenzoate, N-oxypropylenated ethyl p-aminobenzoate, glyceryl p-aminobenzoate; Benzophenones such as e.g. benzophenone-3, benzophenone-4, 2,2',4,4'-tetrahydroxy-benzophenone, 2,2'-dihydroxy-4,4'-dimethoxybenzophenone; Esters of benzalmalonic acid such as e.g. di-(2-ethylhexyl) 4-methoxybenzalmalonate; Organosiloxane compounds carrying chromophore groups such as e.g. polysilicones-15 (PARSOL® SLX by DSM Nutritional Products Ltd), drometrizole trisiloxane (Mexoryl® XL); Salicylate derivatives such as e.g. isopropylbenzyl salicylate, benzyl salicylate, butyl salicylate, ethylhexyl salicylate (PARSOL® EHS by DSM Nutritional Products Ltd), isooctyl salicylate or homomenthyl salicylate (homosalate, PARSOL® HMS by DSM Nutritional Products Ltd); Triazine derivatives such as e.g. ethylhexyl triazone (Uvinul® T-150 by BASF), diethylhexyl butamido triazone (Uvasorb® HEB), bis-ethylhexyloxyphenol methoxyphenyl triazine (Tinosorb® S), and Tris-Biphenyl Triazine (nano) (Tinosorb® A2B by BASF); Benzotriazole derivatives such as e.g. 2,2'-methylene-bis-(6-(2H-benzotriazole-2-yl)-4-(1,1,3,3,-tetramethylbutyl)-phenol (Tinosorb® M by BASF); Encapsulated UV-filters such as e.g. encapsulated ethylhexyl methoxycinnamate (Eusolex® UV-pearls by EMD Chemicals Inc) or microcapsules loaded with UV-filters as e.g. dislosed in EP 1471995; Dibenzoylmethane derivatives such as e.g. 4-tert.-butyl-4'-methoxydibenzoyl-methane (PARSOL® 1789 by DSM Nutritional Products Ltd), dimethoxydibenzoylmethane, isopropyldibenzoylmethane; Amino substituted hydroxybenzophenones such as e.g. 2-(4-diethylamino-2-hydroxy-benzoyl)-benzoic acid hexylester (Aminobenzophenon, Uvinul® A Plus by BASF); Benzoxazol-derivatives such as e.g. 2,4-bis-[5-1(dimethylpropyl)benzoxazol-2-yl-(4-phenyl)-imino]-6-(2-ethylhexyl)-imino-1,3,5-triazine (Uvasorb® K2A by 3V);
In order to enhance the photostability of sun care products it may be desirable to add a photostabilizer. Exemplary photostabilizers known to a skilled person in the art encompass e.g. 3,3-diphenylacrylate derivatives such as e.g. octocrylene (PARSOL® 340 by DSM Nutritional Products Ltd) or Polyester-8 (Polycrylene® by HallStar); Benzylidene camphor derivatives such as e.g. 4-methyl benzylidene camphor (PARSOL® 5000 by DSM Nutritional Products Ltd); Dialkyl naphthalates such as diethylhexyl naphthalate (Corapan® TQ by Symrise) without being limited thereto. An overview on further stabilizers is e.g. given in 'SPF Boosters & Photostability of Ultraviolet Filters', HAPPI, October 2007, p. 77-83 which is included herein by reference. The photostabilizers are generally used in an amount of 0.05 to 10 wt.-% with respect to the total weigh of the topical composition.

Generally, the amount of each UV-filter substance in the topical compositions according to the invention is selected in the range of about 0.1 to 10 wt.-%, preferably in the range of about 0.2 to 7 wt.-%, most preferably in the range of about 0.5 to 5 wt.-% with respect to the total weigh of the topical composition.

The total amount of UVA-filter substance(s), in particular of butyl methoxydibenzoylmethane, in the topical compositions according to the invention is preferable selected in the range of about 0.5 to 8 wt.-% such as e.g. in the range of 2 to 8 wt.-%, in particular in the range of about 1 to 6 wt.-%, such as e.g. in the range of 2.5 to 6 wt.-%, most particular in the range of about 3 to 5 wt.-% such as in the range of with respect to the total weight of the topical composition.

The total amount of UV-filter substances in the topical compositions according to the invention is preferably in the range of about 1 to 40 wt.-%, preferably in the range of about 5 to 30 wt.-%, in particular in the range of 12 to 30 wt.-% such as for example in the range of 20 to 30 wt.-% with respect to the total weight of the topical composition.

Preferred further UVB-filter substances to be used in the topical compositions according to the invention encompass octocrylene, ethylhexyl methoxycinnamate, ethyl hexylsalicylate and/ or homosalate.

Preferred broadband UV-filter substances to be used in the topical compositions according to the invention encompass unsymmetrical s-triazine derivatives such 2,4-Bis-{[4-(2-ethyl-hexyloxy)-2-hydroxy]-phenyl}-6-(4-methoxyphenyl)-1,3,5-triazin, certain benzophenones such as e.g. 2-Hydroxy-4-methoxy-benzophenon, and/ or 2,2'-Methylen-bis-(6-(2H-benzotriazol-2-yl)-4-(1,1,3,3-tetramethyl-butyl)-phenol).

The preferred UVA-filter substance to be used in the topical compositions according to the invention is butyl methoxydibenzoylmethane.

In a particular preferred embodiment the present invention relates to topical compositions according to the invention comprising at least one additional UV-filter selected from the group of butyl methoxydibenzoylmethane, octocrylene, homosalate, 2-phenylbenzimidazol-sulphonic acid or ethylhexyl salicylate as well as mixtures thereof. In this case, it is further preferred if the amount of the microfine double coated titanium dioxide is selected in the range of 2-5 wt.-%, the amount of butyl methoxydibenzoylmethane is selected in the range of 3-5 wt.-%, the amount of octocrylene is selected in the range of 6-15 wt.-%, the amount of homosalate is selected in the range of 2-13 wt.-%, the amount of 2-phenylbenzimidazol-sulphonic acid is selected in the range of 0.5-2 wt.-% and the amount of ethylhexyl salicylate is selected in the range of 2-8 wt.-%, with respect to the total weight of the topical composition. In these compositions the amount of silica beads is furthermore preferably selected in the range of 2 to 4 wt.-% based on the total weight of the composition.

Particular preferred are topical compositions which comprise as UV-filters only microfine double coated titanium dioxide, butyl methoxydibenzoylmethane, octocrylene, homosalate, 2-phenylbenzimidazol-sulphonic acid or titanium dioxide, butyl methoxydibenzoylmethane, octocrylene, homosalate and ethylhexyl salicylate, most preferably in the amounts as indicated in the paragraph above.

In another particular preferred embodiment the present invention relates to topical compositions which comprise as UV-filters only microfine coated zinc oxide and ethylhexyl methoxycinnamate. In this case, it is further preferred if the emulsifier is polyglyceryl-2 dipolyhydroxystearate and the amount of the microfine coated zinc oxide is selected in the range of 5-15 wt.-% and the amount of ethylhexyl methoxycinnamate is selected in the range of 5-12 wt.-%, preferably the amount of the microfine coated zinc oxide is selected in the range of 8-12 wt.-% and the amount of ethylhexyl methoxycinnamate is selected in the range of 6-10 wt.-%, with respect to the total weight of the topical composition. In these compositions the amount of silica beads is furthermore preferably selected in the range of 2 to 4 wt.-% based on the total weight of the composition
The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range of 3 to 10, preferably a pH in the range of 4 to 8 and most preferably a pH in the range of 4 to 7.5. The pH can easily be adjusted as desired with suitable acids such as e.g. citric acid or bases such as sodium hydroxide (e.g. as aqueous solution), Triethanolamine (TEA Care), Tromethamine (Trizma Base) and Aminomethyl Propanol (AMP-Ultra PC 2000) according to standard methods in the art.

The amount of the topical composition to be applied to the skin is not critical and can easily be adjusted by a person skilled in the art. Preferably the amount is selected in the range of 0.1 to 3 mg/ cm² skin, such as preferably in the range of 0.1 to 2 mg/ cm² skin and most preferably in the range of 0.5 to 2 mg / cm² skin.

The following examples are provided to further illustrate the compositions and effects of the present invention. These examples are illustrative only and are not intended to limit the scope of the invention in any way.

The term 'Control' as used in the experimental part refers to formulations without a 'whitening reducer' i.e. without any silica beads.
The term 'reference (Ref)' as used in the experimental part refers to formulations with silica beads not according to the invention (coated silica beads)
The term 'invention (Inv)' as used in the experimental part refers to formulations with silica beads not according to the invention (coated silica beads)

### Example 1:

**Table 1: Formulation**

| **Trade Name** | **INCI Name** | A Control | B (Inv) | C (Ref) |
|---|---|---|---|---|
| | | w% | | |
| PARSOL® 1789 | Butyl methoxydibenzoylmethane | 5.00 | 5.00 | 5.00 |
| DL-alpha-tocopheryl | Tocopheryl acetate | 0.50 | 0.50 | 0.50 |

| acetate | | | | |
|---|---|---|---|---|
| Finsolv TN | C12-15 alkyl benzoate | 5.00 | 5.00 | 5.00 |
| Dermofeel® BGC | Butylene glycol dicaprylate/ dicaprate | 4.00 | 4.00 | 4.00 |
| AMPHISOL® K | Potassium cetyl phosphate | 3.00 | 3.00 | 3.00 |
| Lanette 16 | Cetyl alcohol | 0.80 | 0.80 | 0.80 |
| Lanette O | Cetearyl alcohol | 0.80 | 0.80 | 0.80 |
| Euxyl PE 9010 | Phenoxyethanol; ethylhexylglycerin | 0.80 | 0.80 | 0.80 |
| PARSOL® 340 | Octocrylene | 14.00 | 14.00 | 14.00 |
| PARSOL® HMS | Homosalate | 10.00 | 10.00 | 10.00 |
| Antaron V-220 | VP/Eicosene copolymer | 1.00 | 1.00 | 1.00 |
| Edeta B | Disodium EDTA | 0.10 | 0.10 | 0.10 |
| Keltrol CG-T | Xanthan gum | 0.20 | 0.20 | 0.20 |
| PARSOL® TX | Titanium dioxide; silica; dimethicone | 5.00 | 5.00 | 5.00 |
| Pemulen TR-1 | Acrylates/C10-30 alkyl acrylate crosspolymer | 0.15 | 0.15 | 0.15 |
| | | | | |
| WATER DEM. | Aqua | Ad 100 | Ad 100 | Ad 100 |
| Glycerine 99.5% AMI PH. EUR. Vegetable | Glycerin | 5.00 | 5.00 | 5.00 |
| | | | | |
| Triethanolamine Care | Triethanolamine | 0.25 | 0.25 | 0.25 |
| | | | | |
| PARSOL® HS | Phenylbenzimidazol sulfonic acid | 1.00 | 1.00 | 1.00 |
| Triethanolamine Care | Triethanolamine | 0.80 | 0.80 | 0.80 |
| WATER DEM. | Aqua | 5.00 | 5.00 | 5.00 |
| | | | | |
| Valvance® Touch 210 | Silica | | 3.00 | |
| Valvance® Touch 250 | Silica, methicone | | | 3.00 |

### Sensory evaluation

The samples were prepared as outlined in table 1 and were tested in a blind study with a trained sensorial panel consisting of minimum 8 persons (preferably 12) under the following conditions:
The evaluation takes part on the inner forearm; the panel leader applies 50 µL of the respective sample. Evaluator spreads the product within a defined circle of 5 cm diameter using index or middle finger, circular motion, rate of 2 rotations/second. After 12 rubs the Rub-out Phase continues until a growing resistance signals the termination of this phase. After that point the immediate whitening is rated and after further 20 minutes waiting the whitening-20min-value is assessed. The whitening was quantified on a scale from 0 to 100 in comparison to training standards with known and defined whitening intensities.

**Table 2: Results of the whitening evaluation**

| sample | immediate whitening | % reduction vs control | whitening after 20 min | % reduction vs control |
|---|---|---|---|---|
| A (Control) | 14 | - | 8 | - |
| B (Invention) | 11 | -24% | 5 | -30 % |
| C (Reference) | 12 | -18% | 7 | -8 % |

As can be retrieved from the results depicted in table 2 only the specific uncoated silica beads lead to an overall significantly reduced whitening.

### Example 2:

**Table 3: Formulation**

| | | D Control | F (Inv) | G (Ref) |
|---|---|---|---|---|
| **Trade Name** | **INCI** | **w%** | **w%** | **w%** |
| PARSOL® 1789 | Butyl methoxydibenzoylmethane | 4.00 | 4.00 | 4.00 |
| PARSOL® 340 | Octocrylene | 10.00 | 10.00 | 10.00 |
| PARSOL® EHS | Ethylhexyl salicylate | 5.00 | 5.00 | 5.00 |
| PARSOL® HMS | Homosalate | 4.00 | 4.00 | 4.00 |
| AMPHISOL® K | Potassium cetyl phosphate | 2.50 | 2.50 | 2.50 |
| Lanette O | Cetearyl alcohol | 1.50 | 1.50 | 1.50 |
| Butylated | BHT | 0.05 | 0.05 | 0.05 |
| Hydroxytoluene | | | | |
| Phenonip | Phenoxyethanol; methylparaben; ethylparaben; butylparaben; propylparaben; isobutylparaben | 0.80 | 0.80 | 0.80 |
| Dermofeel® BGC | Butylene glycol dicaprylate/ dicaprate | 8.00 | 8.00 | 8.00 |
| Antaron V-220 | VP/Eicosene copolymer | 0.50 | 0.50 | 0.50 |
| Cetiol CC | Dicaprylyl carbonate | 4.00 | 4.00 | 4.00 |
| Cetiol MM | Myristyl myristate | 0.50 | 0.50 | 0.50 |
| | | | | |
| Glycerin 1,23 (86.5%) Ph. Eur. | Glycerin | 3.00 | 3.00 | 3.00 |
| Edeta BD | Disodium EDTA | 0.10 | 0.10 | 0.10 |
| WATER DEM. | Aqua | Ad 100 | Ad 100 | Ad 100 |
| Pemulen TR-1 | Acrylates/C10-30 alkyl acrylate crosspolymer | 0.15 | 0.15 | 0.15 |
| | | | | |
| Keltrol CG-T | Xanthan gum | 0.15 | 0.15 | 0.15 |
| Parsol® TX | Titanium dioxide; silica; dimethicone | 3.00 | 3.00 | 3.00 |
| | | | | |
| Sodium hydroxid 30% soln. | Aqua; sodium hydroxide | 0.15 | 0.15 | 0.15 |
| Valvance® Touch 210 | Silica | | 3.00 | |
| Valvance® Touch 250 | Silica, methicone | | | 3.00 |

### Sensory evaluation

The samples were prepared as outlined in table 3 and were tested in a blind study with a trained sensorial panel consisting of minimum 8 persons (preferably 12) under the following conditions:
The evaluation takes part on the inner forearm; the panel leader applies 50 µL of the respective sample. Evaluator spreads the product within a defined circle of 5 cm diameter using index or middle finger, circular motion, rate of 2 rotations/second. After 12 rubs the Rub-out Phase continues until a growing resistance signals the termination of this phase. After that point the immediate whitening was rated. The whitening was quantified on a scale from 0 to 100 in comparison to training standards with known and defined whitening intensities.

**Table 4: Results of the whitening evaluation**

| sample | immediate whitening | % reduction vs control |
|---|---|---|
| D (Control) | 6 | - |
| F (Invention) | 5 | -17% |
| G (Reference) | 8 | +33% |

As can be retrieved from the results depicted in table 4 only the specific uncoated silica beads lead to a significantly reduced whitening.

### Example 3:

**Table 5: Formulation**

| | | H Control | K (Inv) |
|---|---|---|---|
| **Tradename** | **INCI** | **Wt.%** | **Wt.-%** |
| Dehymuls PGPH | Polyg lyceryl-2 dipolyhydroxystearate | 5.0 | 5.0 |
| Monomuls 90 O 18 | Glyceryl oleate | 0.5 | 0.5 |
| Finsolv TN | C12-15 Alkyl benzoate | 6.0 | 6.0 |
| Myritol 318 | Caprylic/capric triglyceride | 6.0 | 6.0 |
| PARSOL® MCX | Ethylhexyl methoxycinnamate | 8.0 | 8.0 |
| Myritol 331 | Cocoglycerides | 6.0 | 6.0 |
| Lanette O | Cetearyl alcohol | 1.0 | 1.0 |
| Z-Cote HP1 | Zinc oxide; triethoxycyprylsilane | 10.0 | 10.0 |
| 1,3-Butylenglycol | Butylene glycol | 10.0 | 10.0 |
| Magnesium Sulfate, Heptaphydrate | Magnesium sulfate | 1.0 | 1.0 |
| WATER DEM. | Aqua | Ad 100 | Ad 100 |
| I VALVANCE Touch 210, Lot. 13031201 | Silica | 0.0 | 3.0 |

### Sensory evaluation

The samples were prepared as outlined in table 5 and were tested in a blind study with a trained sensorial panel consisting of 5 persons under the following conditions:
The evaluation takes part on the inner forearm; the panel leader applies 75 µL of the respective sample. Evaluator spreads the product within a defined circle of 5 cm diameter using index or middle finger, circular motion, rate of 2 rotations/second. After 12 rubs the Rub-out phase continues until a growing resistance signals the termination of this phase. After that point the immediate whitening was rated. The whitening was quantified on a scale from 0 to 100 in comparison to training standards with known and defined whitening intensities.

**Table 6: Results of the whitening evaluation**

| sample | Rub-out | % reduction vs control | immediate whitening | % reduction vs control |
|---|---|---|---|---|
| H (Control) | 45 | - | 37 | - |
| K (Invention) | 43 | -5% | 35 | -5 % |

## Claims

1. Topical compositions comprising (1) uncoated, highly porous silica beads having a particle size Dᵥ0 of greater 0.3 µm, a Dᵥ100 of less than 35 µm, a Dᵥ50 selected in the range of 8 to 15 µm determined by a Coulter LS13320 or a Malvern Mastersizer 2000 and an oil absorption capacity selected in the range of 1.2-2.5 cc/g and (2) a microfine double coated titanium dioxide, **characterized in that** the microfine double coated titanium dioxide is of the rutil type and has an inner silica coating and an outer dimethicone coating.

2. The topical composition according to claim 1, **characterized in that** the amount of the silica beads is selected in the range of 0.1 - 5 wt.-% based on the total weight of the cosmetic composition.

3. The topical composition according to claim 1 or 2, **characterized in that** the oil absorption capacity of the silica beads is selected in the range of 1.3 to 1.8 cc/g.

4. The topical composition according to claims 1 to 3, **characterized in that** the amount of the microfine double coated titanium dioxide is selected in the range of 0.5 to 15 wt.-%.

5. The topical composition according to any one of claims 1 to 4, **characterized in that** the primary particle size (w/o any coating) of the titanium dioxide is selected in the range of 2 to 100 nm, and the secondary particle size (with double coating) is selected in the range of 0.05 and 50 µm.

6. The topical composition according to any one of claims 1 to 5, **characterized in that** the primary particle size of the titanium dioxide is selected in the range of 5 to 50 nm and the secondary particle size is selected in the range of 0.1 and 1 µm.

7. The topical composition according to any one of claims 1 to 6, **characterized in that** the composition further comprises at least one additional UV-filter selected from the group of butyl methoxydibenzoylmethane, octocrylene, homosalate, 2-phenylbenzimidazol-sulphonic acid, ethylhexyl methoxycinnamate and ethyl hexyl salicylate as well as mixtures thereof.

8. The topical composition according to any one of claims 1 to 7, **characterized in that** the topical composition is in the form of an oil-in-water (O/W) emulsion comprising an oily phase dispersed in an aqueous phase in the presence of an O/W emulsifier or a W/O emulsions comprising an aqueous phase dispersed in an oily phase in the presence of a W/O emulsifier.

9. The topical composition according to claim 8, **characterized in that** the O/W emulsifier is potassium cetyl phosphate and the W/O emulsifier is Polyglyceryl-2 Dipolyhydroxystearate.

10. Use of uncoated, highly porous silica beads having a particle size Dᵥ0 of greater than 0.3 µm, a Dᵥ100 of less than 35 µm, a Dᵥ50 selected in the range of 8 to 15 µm determined by a Coulter LS 13320 or a Malvern Mastersizer 2000 and an oil absorption capacity selected in the range of 1.2-2.5 cc/g to reduce the whitening effect of a microfine double coated titanium dioxide in a topical composition, **characterized in that** the microfine double coated titanium dioxide is of the rutil type and has an inner silica coating and an outer dimethicone coating.

11. A method of reducing the whitening effect of a microfine double coated titanium dioxide, **characterized in that** the microfine double coated titanium dioxide is of the rutil type and has an inner silica coating and an outer dimethicone coating. in a topical composition, said method comprising the step of adding to the topical composition uncoatedsilica beads having a particle size Dᵥ0 of greater than 0.3 µm, a Dᵥ100 of less than 35 µm, a Dᵥ50 selected in the range of 8 to 15 µm determined by a Coulter LS 13320 or a Malvern Mastersizer 2000 and an oil absorption capacity selected in the range of 1.2-2.5 cc/g and appreciating the effect.

## Patentansprüche

1. Topische Zusammensetzungen umfassend (1) unbeschichtete, hochporöse Silicakügelchen mit einer Partikelgröße Dᵥ0 von größer als 0,3 µm, einem Dᵥ100 von kleiner als 35 µm, einem Dᵥ50 ausgewählt in dem Bereich von 8 bis 15 µm, bestimmt durch ein Coulter LS 13320 oder ein Malvern Mastersizer 2000, und einer Ölabsorptionskapazität ausgewählt in dem Bereich von 1,2-2,5 cc/g und (2) ein mikrofeines, doppelt beschichtetes Titandioxid, **dadurch gekennzeichnet, dass** das mikrofeine, doppelt beschichtete Titandioxid vom Rutiltyp ist und eine innere Silicabeschichtung und eine äußere Dimethiconbeschichtung aufweist.

2. Topische Zusammensetzung gemäß Anspruch 1, **dadurch gekennzeichnet, dass** die Menge der Silicakügelchen in dem Bereich von 0,1-5 Gew.-% bezogen auf das Gesamtgewicht der kosmetischen Zusammensetzung ausgewählt ist.

3. Topische Zusammensetzung gemäß Anspruch 1 oder 2, **dadurch gekennzeichnet, dass** die Ölabsorptionskapazität der Silicakügelchen in dem Bereich von 1,3 bis 1,8 cc/g ausgewählt ist.

4. Topische Zusammensetzung gemäß Ansprüchen 1 bis 3, **dadurch gekennzeichnet, dass** die Menge des mikrofeinen, doppelt beschichteten Titandioxids in dem Bereich von 0,5 bis 15 Gew.-% ausgewählt ist.

5. Topische Zusammensetzung gemäß einem der Ansprüche 1 bis 4, **dadurch gekennzeichnet, dass** die Primärpartikelgröße (ohne jede Beschichtung) des Titandioxids in dem Bereich von 2 bis 100 nm ausgewählt ist und die Sekundärpartikelgröße (mit doppelter Beschichtung) in dem Bereich von 0,05 und 50 µm ausgewählt ist.

6. Topische Zusammensetzung gemäß einem der Ansprüche 1 bis 5, **dadurch gekennzeichnet, dass** die Primärpartikelgröße des Titandioxids in dem Bereich von 5 bis 50 nm ausgewählt ist und die Sekundärpartikelgröße in dem Bereich von 0,1 und 1 µm ausgewählt ist.

7. Topische Zusammensetzung gemäß einem der Ansprüche 1 bis 6, **dadurch gekennzeichnet, dass** die Zusammensetzung ferner wenigstens einen zusätzlichen UV-Filter ausgewählt aus der Gruppe von Butylmethoxydibenzoylmethan, Octocrylen, Homosalat, 2-Phenylbenzimidazolsulfonsäure, Ethylhexylmethoxycinnamat und Ethylhexylsalicylat sowie Gemischen davon ausgewählt ist.

8. Topische Zusammensetzung gemäß einem der Ansprüche 1 bis 7, **dadurch gekennzeichnet, dass** die topische Zusammensetzung in der Form einer Öl-in-Wasser(O/W)-Emulsion, die eine Ölphase umfasst, die in Gegenwart eines O/W-Emulgators in einer wässrigen Phase dispergiert ist, oder einer W/O-Emulsion, die eine wässrige Phase umfasst, die die in Gegenwart eines W/O-Emulgators in einer öligen Phase dispergiert ist, vorliegt.

9. Topische Zusammensetzung gemäß Anspruch 8, **dadurch gekennzeichnet, dass** der O/W-Emulgator Kaliumcetylphosphat ist und der W/O-Emulgator Polyglyceryl-2-dipolyhydroxystearat ist.

10. Verwendung von unbeschichteten, hochporösen Silicakügelchen mit einer Partikelgröße Dᵥ0 von größer als 0,3 µm, einem Dᵥ100 von kleiner als 35 µm, einem Dᵥ50 ausgewählt in dem Bereich von 8 bis 15 µm, bestimmt durch ein Coulter LS 13320 oder ein Malvern Mastersizer 2000, und einer Ölabsorptionskapazität ausgewählt in dem Bereich von 1,2-2,5 cc/g zum Verringern der weißmachenden Wirkung eines mikrofeinen, doppelt beschichteten Titandioxids in einer topischen Zusammensetzung, **dadurch gekennzeichnet, dass** das mikrofeine, doppelt beschichtete Titandioxid vom Rutiltyp ist und eine innere Silicabeschichtung und eine äußere Dimethiconbeschichtung aufweist.

11. Verfahren zum Verringern der weißmachenden Wirkung eines mikrofeinen, doppelt beschichteten Titandioxids, **dadurch gekennzeichnet, dass** das mikrofeine, doppelt beschichtete Titandioxid vom Rutiltyp ist und eine innere Silicabeschichtung und eine äußere Dimethiconbeschichtung aufweist, in einer topischen Zusammensetzung, wobei das Verfahren den Schritt des Zugebens von nichtbeschichteten Silicakügelchen mit einer Partikelgröße Dᵥ0 von größer als 0,3 µm, einem Dᵥ100 von kleiner als 35 µm, einem Dᵥ50 ausgewählt in dem Bereich von 8 bis 15 µm, bestimmt durch ein Coulter LS 13320 oder ein Malvern Mastersizer 2000, und einer Ölabsorptionskapazität ausgewählt in dem Bereich von 1,2-2,5 cc/g, zu der topischen Zusammensetzung und Wahrnehmen der Wirkung umfasst.

## Revendications

1. Compositions topiques comprenant (1) des billes de silice hautement poreuses et non revêtues, ayant une taille de particules Dᵥ0 supérieure à 0,3 µm, une Dᵥ100 inférieure à 35 µm, une Dᵥ50 choisie dans la plage allant de 8 à 15 µm, déterminée à l'aide d'un Coulter LS13320 ou d'un Malvern Mastersizer 2000, et une capacité d'absorption d'huile choisie dans la plage de 1,2-2,5 cc/g et (2) un dioxyde de titane microfin à double revêtement, **caractérisé en ce que** le dioxyde de titane microfin à double revêtement est de type rutile et possède un revêtement de silice interne et un revêtement de diméthicone externe.

2. Composition topique selon la revendication 1, **caractérisée en ce que** la quantité des billes de silice est choisie dans la plage de 0,1-5% en poids sur la base du poids total de la composition cosmétique.

3. Composition topique selon la revendication 1 ou 2, **caractérisée en ce que** la capacité d'absorption d'huile des billes de silice est choisie dans la plage allant de 1,3 à 1,8 cc/g.

4. Composition topique selon les revendications 1 à 3, **caractérisée en ce que** la quantité de dioxyde de titane microfin à double revêtement est choisie dans la plage allant de 0,5 à 15% en poids.

5. Composition topique selon l'une quelconque des revendications 1 à 4, **caractérisée en ce que** la taille de particules primaires (sans aucun revêtement) du dioxyde de titane est choisie dans la plage allant de 2 à 100 nm, et la taille de particules secondaires (avec un double revêtement) est choisie dans la plage comprise entre 0,05 et 50 µm.

6. Composition topique selon l'une quelconque des revendications 1 à 5, **caractérisée en ce que** la taille de particules primaires du dioxyde de titane est choisie dans la plage allant de 5 à 50 nm, et la taille de particules secondaires est choisie dans la plage comprise entre 0,1 et 1 µm.

7. Composition topique selon l'une quelconque des revendications 1 à 6, **caractérisée en ce que** la composition comprend en outre au moins un filtre anti-UV supplémentaire choisi dans le groupe constitué par le butylméthoxydibenzoylméthane, l'octocrylène, l'homosalate, l'acide 2-phénylbenzimidazolsulfonique, le méthoxycinnamate d'éthylhexyle et le salicylate d'éthylhexyle, ainsi que des mélanges de ceux-ci.

8. Composition topique selon l'une quelconque des revendications 1 à 7, **caractérisée en ce que** la composition topique se trouve sous la forme d'une émulsion huile-dans-eau (H/E) comprenant une phase huileuse dispersée dans une phase aqueuse en présence d'un émulsifiant H/E ou d'une émulsion E/H comprenant une phase aqueuse dispersée dans une phase huileuse en présence d'un émulsifiant E/H.

9. Composition topique selon la revendication 8, **caractérisée en ce que** l'émulsifiant H/E est le cétylphosphate de potassium et l'émulsifiant E/H est le 2-dipolyhydroxystéarate de polyglycérol.

10. Utilisation de billes de silice hautement poreuses et non revêtues, ayant une taille de particules Dᵥ0 supérieure à 0,3 µm, une Dᵥ100 inférieure à 35 µm, une Dᵥ50 choisie dans la plage allant de 8 à 15 µm, déterminée à l'aide d'un Coulter LS13320 ou d'un Malvern Mastersizer 2000, et une capacité d'absorption d'huile choisie dans la plage de 1,2-2,5 cc/g, afin de réduire l'effet de blanchiment de dioxyde de titane microfin à double revêtement dans une composition topique, **caractérisée en ce que** le dioxyde de titane microfin à double revêtement est de type rutile et possède un revêtement de silice interne et un revêtement de diméthicone externe.

11. Méthode de réduction de l'effet de blanchiment d'un dioxyde de titane microfin à double revêtement, **caractérisée en ce que** le dioxyde de titane microfin à double revêtement est de type rutile et possède un revêtement de silice interne et un revêtement de diméthicone externe, dans une composition topique, ladite méthode comprenant l'étape consistant à ajouter à la composition topique des billes de silice non revêtues, ayant une taille de particules Dᵥ0 supérieure à 0,3 µm, une Dᵥ100 inférieure à 35 µm, une Dᵥ50 choisie dans la plage allant de 8 à 15 µm, déterminée à l'aide d'un Coulter LS13320 ou d'un Malvern Mastersizer 2000, et une capacité d'absorption d'huile choisie dans la plage de 1,2-2,5 cc/g, et à apprécier l'effet.
